# EUROPEAN PATENT SPECIFICATION

(11) **EP 2 349 455 B1**
(45) Date of publication and mention of the grant of the patent: **18.01.2017**
(21) Application number: 09826561.4
(22) Date of filing: 02.11.2009
(51) Int. Cl.: H03H 7/01, A61N 1/05, A61N 1/08, H03H 1/00

(54) **LEAD EXTENSION**
LEITERVERLÄNGERUNG
RALLONGE DE CÂBLE

(30) Priority: 31.10.2008 US 110016 P
(43) Date of publication of application: 03.08.2011
(73) Proprietor: QIG Group, LLC, Clarence, New York 14031 (US)
(72) Inventor: SWOYER, John, Andover Minnesota 55304 (US); YE, Qingshan, Plymouth Minnesota 55441 (US)
(74) Representative: Schwabe - Sandmair - Marx
(86) International application number: PCT/US2009/062934
(87) International publication number: WO 2010/056558

(56) References cited:
- US-A1- 2003 009 207
- US-A1- 2008 132 987
- US-A1- 2008 161 886
- US-B1- 6 324 433
- US-B2- 7 363 090

## Description

### BACKGROUND

MRI systems are well known to those skilled in the art and are well described in U.S. Patent No. 7,363,090 (Halperin et al.). There are three types of electromagnetic fields used in an MRI unit. The first type is the main static magnetic field designated B₀ which is used to align protons in body tissue. The field strength varies from 0.5 to 3.0 Tesla in most of the currently available MRI units in clinical use. Some of the newer MRI system fields can go as high as 4 to 5 Tesla. It is a basic principle of physics that a magnetic field must either be time-varying as it cuts across the conductor, or the conductor itself must move within the magnetic field for currents to be induced. Thus, it is not likely (other than sudden system shut down) that the static MRI magnetic field can induce currents into a lead wire system.

The second type of field produced by magnetic resonance imaging is the pulsed RF field which is generated by the body coil or head coil. This is used to change the energy state of the protons and illicit MRI signals from tissue. The RF field is homogeneous in the central region and has two main components: (1) the magnetic field is circularly polarized in the actual plane; and (2) the electric field is related to the magnetic field by Maxwell's equations. In general, the RF field is switched on and off during measurements and usually has a frequency of 21 MHz to 64 MHz to 128 MHz depending upon the static magnetic field strength. The frequency of the RF pulse varies with the field strength of the main static field where: RF PULSED FREQUENCY in MHz= (42.56) (STATIC FIELD STRENGTH IN TESLA).

The third type of electromagnetic field is the time-varying magnetic gradient fields designated B₁ which are used for spatial localization. These change their strength along different orientations and operating frequencies on the order of 1 kHz. The vectors of the magnetic field gradients in the X, Y and Z directions are produced by three sets of orthogonally positioned coils and are switched on only during the measurements. In some cases, the gradient field has been shown to elevate natural heart rhythms (heart beat). This is not completely understood, but it is a repeatable phenomenon. The gradient field is not considered by many researchers to create any other adverse effects.

At the frequencies of interest in MRI, RF energy can be absorbed and converted to heat. The power deposited by RF pulses during MRI is complex and dependent upon the power (Specific Absorption Rate (SAR) Level) and duration of the RF pulse, the transmitted frequency, the number of RF pulses applied per unit time, and the type of configuration of the RF transmitter coil used. The amount of heating also depends upon the volume of tissue imaged, the electrical resistivity of tissue and the configuration of the anatomical region imaged. There are also a number of other variables that depend on the placement in the human body of the IPG and its associated lead wire(s). The routing of the lead and the lead length are also very critical as to the amount of induced current and heating that would occur.

The cause of heating in an MRI environment is twofold: (a) RF field coupling to the lead can occur which induces significant local heating; and (b) currents induced between the distal TIP and tissue during MRI RF pulse transmission sequences can cause local Ohms Law heating in tissue next to the distal TIP electrode of the implanted lead. The RF field of an MRI scanner can produce enough energy to induce lead wire currents sufficient to destroy some of the adjacent tissue. Tissue ablation has also been observed in pacing applications. The effects of this heating are not readily detectable by monitoring during the MRI.

PAD electrodes are very common in neurostimulator applications. For example, spinal cord stimulators or deep brain stimulators can include a plurality of PAD electrodes to make contact with nerve tissue. A good example of this also occurs in a cochlear implant. In a typical cochlear implant there would be sixteen RING electrodes that the physician places by pushing the electrode up into the cochlea. Several of these RING electrodes make contact with auditory nerves.

As previously mentioned, just variations in the lead wire length can significantly affect how much heat is generated. If one assumes, for example, a 3.0 Tesla MRI system, which would have an RF pulsed frequency of 128 MHz, there are certain implanted lead lengths that would couple efficiently as fractions of the 128 MHz wavelength. It is typical that a hospital will maintain an inventory of various leads and that the implanting physician will make a selection depending on the size of the patient, implant location and other factors. Accordingly, the implanted or effective lead wire length can vary considerably. There are certain implanted lead wire lengths that just do not couple efficiently with the MRI frequency and there are others that would couple very efficiently and thereby produce the worst case for heating.

Magnetic Resonance Imaging (MRI) systems are used to visualize internal body systems to a degree previously impossible. The use of MRI is contraindicated for many implanted electrical leads, as the leads may act as antennas, picking up the RF energy and converting that energy into heat at the distal end of the lead having the exposed electrodes in contact with the tissue. Some leads have been developed which have band stop filters (BSFs) included in the leads near the electrodes. The BSFs are provided to reduce the likelihood of heating tissue during an MRI procedure, making such procedures available to patients having implanted electrical devices. Such devices include pacemakers, cardioverters, and neurological stimulation devices. The size of the BSFs is often not an issue, due to the intended target site, the small number of lead wires, and/or the implantation techniques.

Deep Brain Stimulation (DBS) can be used to stimulate brain tissue deep within the brain. DBS can be used to treat Parkinson's disease, essential tremor, and epilepsy. The small diameter, fine stimulation leads would have to be substantially increased in profile to include BSFs. This increased profile would have to be pushed through the brain tissue to the target site. Such much larger stimulation leads would require substantially larger cannulas to advance the leads within. The larger cannulas would also have to be advanced through and deep into the brain. The larger size is undesirable and for these reasons the BSFs are not included in DBS leads.

Document US 2008 0,132,987 relates to medical lead systems utilizing electromagnetic bandstop filters, which can be utilized in a magnetic resonance imaging (MRI) environment for patients who have implanted medical devices. Such lead systems may be advantageously used in left ventricle cardiac stimulation systems, neuro-stimulation systems, and deep brain electrodes used for the treatment of Parkinson's disease and other movement disorders. The band-stop filters, which include a tuned parallel capacitor and inductor circuit, are backwards compatible with known implantable deployment systems.

Spinal Cord Stimulation (SCS) can be used to stimulate spinal cord tissue, typically from the epidural space. The SCS lead is typically introduced through a needle into the epidural space, and the needle removed over the lead once in place. Modern SCS leads may have several, small diameter conductor wires contained within the lead body. The small diameter SCS leads would have to be substantially increased in profile to include BSFs. This increased profile would have to be pushed through the needle and epidural space, sometimes through a curved path, to the target site. Such much larger stimulation leads would require substantially larger needles and leads if having BSFs included in the lead body. The larger leads and needles are undesirable, and for this reason, the BSFs are also not included in SCS leads. SCS is well known in the art, and is discussed in U.S. Patent No. 6,233,488.

When an MRI would be beneficial to, and given to most patients in a given medical situation, it may be denied to patients having DBS or SCS leads implanted. The treating physician may not want to incur the risk of heating the neurological tissue. The physician may balance the risk of tissue heating against the risk of not using the MRI, depending on severity of the medical reason for performing the MRI. The risk of possible tissue heating may not be worth the gain from the MRI.

The risk assessment is difficult, as the lead placement, travel path, length, orientation within the body relative to the MRI fields, and the nature of the tissue can all play a role in determining whether and how much tissue heating will occur. MRIs have been performed on DBS and SCS patients with no apparent ill effects. MRIs have been denied DBS and SCS patients due to the fear of ill effects.

What would be desirable are devices and methods providing deep brain stimulation and spinal cord stimulation while using MRI safe leads.

### SUMMARY

The present invention relates to a lead extension for coupling to a lead, the lead including a lead distal region having at least one electrode thereon, a lead proximal region having at least one electrical contact thereon, and a lead length, the lead extension comprising: a lead extension body having a lead extension proximal region, a lead extension distal region, and at least one electrical conductor disposed within and extending between the lead extension proximal and distal regions; at least one band stop filter (BSF) including a capacitor in parallel with an inductor, the parallel capacitor and inductor combination placed in series with each of the electrical conductors somewhere between the lead extension proximal and distal regions, wherein values of capacitance and inductance have been selected such that the band stop filter is resonant around a selected frequency; wherein the lead extension includes a distal connector and in which the BSF is included within the lead extension distal connector, wherein the lead extension distal connector includes at least one insertion port for receiving the lead proximal region and in which the BSF is coupled to the insertion port.

Disclosed is also a method of placing a neurostimulation lead. Said method is not claimed in the claims.

Some embodiments of the present invention provide lead extensions, systems, and methods for MRI compatible deep brain stimulation (DBS) systems and spinal cord stimulation (SCS) systems. Lead extensions are provided having band stop filters (BSFs) which resonate at a frequency expected from MRI systems to create a very high impedance which can effectively decouple the implanted lead from the lead extension proximal of the BSF and change the effective length. Changing the effective length can reduce the likelihood of undesirably heating tissue near the DBS or SCS electrodes during MRI. Some lead extensions include BSFs in a distal connector for coupling to the lead contacts. The BSFs can be included within a burr hole cap base which can also include a connector for connecting to the DBS lead. DBS and SCS leads having a sacrificial proximal portion and intermediately disposed electrical contacts are also provided.

Some embodiments of the present invention provide resonant tank or band stop filters which can be placed at various locations along the active implantable medical device lead extension system, which also prevents current from circulating at selected frequencies of the medical therapeutic device. Some embodiment tank filters are designed to resonate at 64 MHz for use in an MRI system operating at 1.5 Tesla (or 128 MHz for a 3 Tesla system).

Some embodiments of the present invention include lead extensions for coupling to a lead where the lead has a lead distal region having at least one electrode thereon, a lead proximal region having at least one electrical contact thereon, and a lead length. The lead extension can include a lead extension body having a lead extension proximal region, a lead extension distal region, and at least one electrical conductor disposed within and extending between the lead extension proximal and distal regions. The lead extension can also have at least one band stop filter (BSF) including a capacitor in parallel with an inductor, with the parallel capacitor and inductor combination placed in series with each (or each and every one) of the electrical conductors somewhere between the lead extension proximal and distal regions. The values of capacitance and inductance can be selected such that the band stop filter is resonant around a selected frequency, for example the expected frequency of the MRI system to be encountered, for example, 64 MHz or 128 MHz.

In some such lead extensions, the BSF has a Q factor, and the overall Q of the BSF is selected to balance impedance at the selected frequency versus frequency band width characteristics. The BSF can be located a distance from the lead extension distal region adapted to reduce resonance with the MRI signal when used in combination with the lead length, in some embodiments. The distance from the BSF to the lead distal end when the lead and the lead extension are coupled together is substantially not equal to a fractional wavelength 1/n of the MRI system, where n is between 1 and 4. In one example, the distance from the BSF to the lead distal end when the lead and the lead extension are coupled together is less than about 15 cm. The lead extension can include a distal connector and the BSF can be included within the lead extension distal connector. The lead extension distal connector can include at least one insertion port for receiving the lead proximal region where the BSF is coupled to the insertion port.

In some lead extensions, the lead extension distal region includes a burr hole cap base for capping a burr hole, the burr hole cap base including an annular body disposed between a central aperture and an outer periphery, the body being MRI compatible. The burr hole cap base can also include at least one connector coupled to the body for electrically coupling to the lead proximal contact, wherein the BSF is mechanically coupled to the burr hole cap base.

Some embodiments of the present invention provide a lead for neuro stimulation (NS), the lead including a distal end and a proximal end having a first length therebetween, and a distal region having at least one electrode adapted for NS thereon. The lead can also have an intermediate region having at least one electrical contact thereon and disposed between the lead distal end and the lead proximal end, where a second length exists between the intermediate region and the lead distal end. At least one electrical conductor can extend between and be in electrical communication with the electrode and the electrical contact, where the first length is at least about twice the second length, and wherein the lead can be severed proximal of the electrical contact without compromising the NS functionality.

In some such leads, the intermediate region includes a visual indicia disposed proximal of the electrical contact indicating a location for cutting the lead after implantation. The intermediate region can include a region of preferential weakness disposed proximal of the contact indicating a location for severing the lead after implantation, where the region of preferential weakness may include a circumferential groove or scoring. Some leads have an outer diameter of less than about 2 mm and the first length is less than about 15 cm. The lead may have at least about 4 distinct conductor wires and an outer diameter of less than about 2 mm.

Some embodiments of the invention provide a burr hole cap base for capping a burr hole, the burr hole cap base including an annular body disposed between a central aperture and an outer periphery, the body being MRI compatible. The base can have at least one connector coupled to the body for electrically coupling to at least one proximal contact of a DBS lead and at least one BSF electrically coupled to the at least one connector, the BSF including a capacitor in parallel with an inductor. Some bases include a lead feed through for feeding the lead from the central aperture to the outer periphery. The base can include a lead fixation structure for fixing position of the lead and inhibiting lead movement after fixation. The base body can include a lead feed through fixation structure for feeding the lead from the central aperture to the outer periphery and preventing movement of the lead at the feed through.

Also described are methods for placing a neurological stimulation (NS) lead, some methods including advancing a tubular introducer to near a target tissue, advancing the NS lead to near the target tissue within the tubular introducer, and removing the tubular introducer over the NS lead. The methods can also include coupling a NS electrical contact to a lead extension distal electrical connector, in which the lead extension includes a band stop filter (BSF) in series with a lead extension electrical conductor.

In some such methods, the target tissue is brain tissue, the NS lead is a deep brain stimulation (DBS) lead, and the tubular introducer is a cannula. The lead extension electrical connector can be disposed within a burr hole cap base, such that the DBS lead extends through the burr hole cap base and the DBS lead proximal region couples to the burr hole cap base. In other such methods, the target tissue is spinal cord nerve tissue, the NS lead is a spinal cord stimulation (SCS) lead, and the tubular introducer is a hollow needle.

Some methods further include severing the NS lead proximal of the lead electrical contact after the tubular introducer has been removed over the NS lead. The length removed can be longer than the length remaining, in some methods. The lead extension can be configured such that the length from the BSF to the lead electrode is not near a substantial resonant wavelength of an expected MRI system, or is less than that wavelength, in various embodiments. Methods can also include electrically coupling a lead extension proximal region to a pulse generator. Performing an MRI including the target tissue after the lead extension with BSF has been coupled to the inserted lead is included in some methods. The MRI is performed prior to connecting the lead extension to an IPG in some embodiment methods. The target tissue can also be a peripheral nerve selected from the group consisting of a sacral nerve, occipital nerve, facial nerve, hypoglossal nerve, vagus nerve, and splanchnic nerve.

In various methods, the lead extension is configured such that the length from the BSF to the lead electrode is less than about 20 cm, 15 cm, 12 cm, and 10 cm. The lead extension is configured such that the length from the BSF to the lead electrode is less than about one-half wavelength or one-quarter wavelength of the expected MRI system. If the wavelength is about 46 cm (e.g. a 1.5 Tesla system), then the lengths would be less than about 23 and less than about 11-12 cm, respectively. If the wavelength is about 23 cm (e.g. a 3 Tesla system), the lengths would be less than about 11-12 cm, and less than about 5-6 cm, respectively.

Some embodiments of the present invention provide resonant tank or band stop filters which can be placed at various locations along the active implantable medical device lead extension system. These band stop filters prevent current from circulating at selected frequencies of the medical therapeutic device. For example, for an MRI system operating at 1.5 Tesla, the pulse RF frequency is 64 MHz (or 128 MHz for a 3 Tesla system). The band stop filters of the present invention can be designed to resonate at 64 MHz and thus create an open circuit in the implanted lead wire system at that selected frequency.

In some embodiments, the overall Q factor of the band stop filter is selected to balance impedance at the selected frequency versus frequency band width characteristics. More specifically, the Q of the inductor is relatively maximized and the Q of the capacitor is relatively minimized to reduce the overall Q of the band stop filter. The Q of the inductor is relatively maximized by minimizing the parasitic resistive loss in the inductor, and the Q of the capacitor is relatively minimized by raising its equivalent series resistance (ESR) of the capacitor (or by adding resistance or a resistive element in series with the capacitor element of the bank stop tank filter). This reduces the overall Q of the band stop filter in order to broaden its 3 dB points and thereby attenuate current flow through the lead wire along a range of selected frequencies. In IPG or external medical device applications, the range of selected frequencies includes a plurality of MRI pulsed frequencies. The capacitor Q can be raised in a controlled manner in the tank filter circuit in order to adjust its Q and adjust the band stop frequency width in the range of MRI pulsed frequencies.

The overall Q of the tank filter circuit may be reduced by increasing the Q of the inductor and reducing the Q of the capacitor. In this regard, minimizing resistive loss in the inductor maximizes the Q of the inductor, and raising the equivalent series resistance of the capacitor minimizes the Q of the capacitor. The net effect is to reduce the overall Q of the tank filter circuit which widens the band stop width to attenuate current flow through the lead wire along a range of selected frequencies. As discussed herein, the range of selected frequencies may include a plurality of MRI pulse frequencies.

Some of the embodiments described herein are also applicable to a wide range of other active implantable medical devices, including deep brain stimulators, spinal cord stimulators, cochlear implants, ventricular assist devices, artificial hearts, drug pumps, and the like. Some embodiments of the present invention can fulfill needs regarding reduction or elimination of undesirable currents and associated heating in implanted lead wire systems.

### DESCRIPTION OF DRAWINGS

FIG 1 is a transverse, cross-sectional view of a human head having a deep brain stimulation (DBS) lead implanted within, extending out through a burr hole cap, and coupled to an implantable pulse generator (IPG) through a lead extension having a band stop filter (BSF).
FIG 2 is a schematic diagram of a two conductor lead extension connector having two band stop filters within.
FIG 3 is a schematic diagram of two conductors having one band stop filter per lead conductor.
FIG 4 is a side view of a stimulation lead having distal electrodes, intermediate contacts, and a removable proximal portion.
FIG 5 is a fragmentary, perspective view of a lead extension distal connector receiving a lead proximal region, with the lead extension distal connector having band stop filters within.
FIG 6 is a fragmentary, transverse, cross-sectional view of the lead extension connector and lead of FIG 5, showing the band stop filters within.
FIG 7 is a highly diagrammatic top view of a burr hole cap base and lead extension connector having set screw blocks and band stop filters within, receiving a DBS lead proximal contact carrying region.

### DETAILED DESCRIPTION

The embodiments described in this present application are intended to illustrate varies embodiments of the present invention and are not intended to limit the invention to those embodiments. The limitations are recited in the claims.

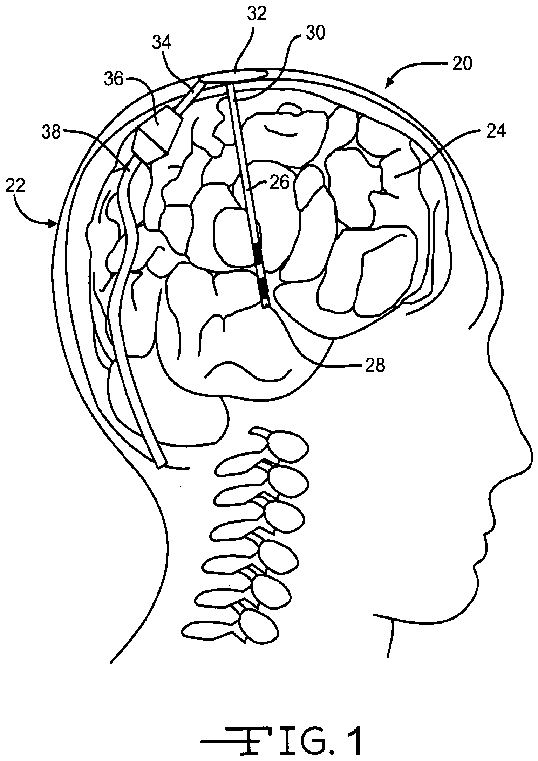

FIG 1 illustrates a human patient 20 having a skull 22 and a brain 24 within. A deep brain stimulation (DBS) lead 26 is shown inserted into brain 24 for a deep brain stimulation therapy. DBS lead 26 includes a lead distal end 28 having two electrodes in the example shown in FIG 1. DBS lead 26 is shown exiting the skull at 30 through a burr hole covered by a burr hole cap 32. DBS lead 26 continues proximally to a DBS lead proximal region 34 which is coupled to a lead extension connector 36. In the example shown, lead extension connector 36 is coupled to a lead extension distal region 38. In many applications, DBS lead proximal region 34, lead extension connector 36, and lead extension distal region 38 are all tunneled outside of the skull but underneath the skin. In some applications, the lead extension can be tunneled down the neck and to an implantable pulse generator (IPG) for the deep brain stimulation therapy.

DBS implantation methods are well known to those skilled in the art. One explanation may be found in U.S. Patent Application Publication No. 2007/0233158. While methods for implanting DBS lead 26 need not be explained in great detail, a brief summary may be useful here. Prior to implantation, standard visualization methods including MRI's, X-rays, CAT scans, and the like can be performed. Often radiopaque markers called fuduciaries are secured to the patient's skull to provide fixed points of reference which will show up on the early visualization procedures and allow accurate points of reference and navigation during the actual implantation procedure. The location of the target region can be mapped out and the route planned ahead of time, using the results from the visualization procedures.

A stereotactic frame is typically secured to the patient's head, to fix movement of the devices relative to the head. A burr hole can be drilled through the skull, providing access to the brain for the DBS lead and other instruments. A burr hole cap base may then be put in place over the burr hole. In some methods, a small micro recording lead can be advanced to the target site and used to record brain signals to determine the proper location for implanting the DBS lead. The micro recording lead is often advanced within a stiffer and more pushable micro cannula and the micro recording lead pushed past the distal end of the cannula into the target tissue.

With the target tissue located, the micro recording lead can be withdrawn and replaced with a macro stimulation lead in some methods. The macro stimulation lead can also be advanced within a stiffer, more pushable macro cannula. The macro stimulation lead can be used to test stimulation of the target tissue. With the location better identified and partially tested, the macro cannula and macro lead can be withdrawn.

The stereotactic equipment can be used to advance a cannula over a desired path to the target tissue location. DBS lead 26 can be advanced together and/or within the cannula to approach the target tissue site. The DBS lead may have multiple wire conductors but yet preferably have a small profile to avoid displacing brain tissue as much as possible. Given the small desired size of the DBS lead, as previously mentioned, it is often undesirable to make the cannula and lead large enough to advance the band stop filters through the cannula to near the target tissue site. With the DBS lead implanted, the cannula can be withdrawn over the DBS lead, leaving the lead in place. The free, proximal end of the DBS lead carrying the contact electrodes also must fit through the cannula in most applications. With the free end of the DBS lead accessible, the lead proximal region may exit the burr hole through a central opening in a burr hole cap 32. DBS lead proximal region 34 can then be electrically and mechanically coupled to a connector 36 which is electrically and mechanically coupled to the lead extension body. In some embodiments, band stop filters are included within the distal connector of the lead extension. In other embodiments, the band stop filters can be incorporated within the lead extension body itself, as the lead extension body does not have to be small enough to pass through the cannula. The lead extension body can be further tunneled under the scalp, down the neck, and to an implantable pulse generator (IPG) which can be located in a surgically formed pocket.

Burr hole cap 32 often includes an annular base portion which covers the edges of the burr hole itself, as well as a cap which fits into the central opening of the burr hole cap base, while allowing the lead to be fed through the burr hole base and/or cap.

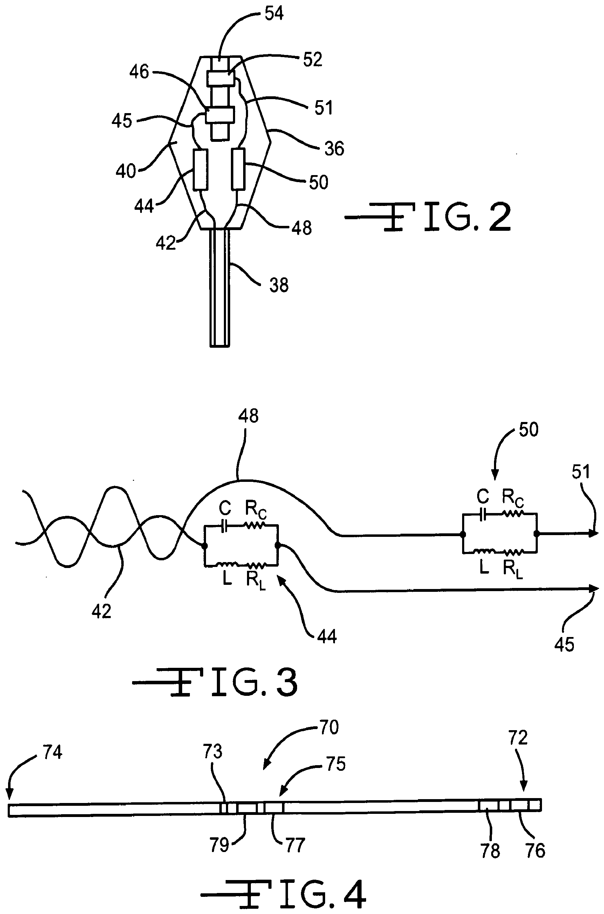

FIG 2 shows the lead extension connector 36 and lead extension distal region 38 of FIG 1. Lead extension connector 36 is shown in a highly diagrammatic, cutaway fashion. Lead extension 36 includes a body 40, which can be formed of an MRI compatible material, for example, a plastic. In the example shown, the connector is adapted to couple two extension conductor wires to two conductor wires within the lead, which presumably has two distal electrodes. Lead extension 36 includes a first conductor or wire 42 coupled to a first band stop filter 44 which is coupled to a first receiving contact 46 through a first conducting wire 45. Similarly, a second conductor or wire 48 is coupled to a second band stop filter 50 which is coupled to a second receiving contact 52 through a conducting wire 51.

In the example shown, receiving contacts 46 and 52 form compressive metallic rings which are urged against a lead carrying external ring or band contacts as carried on the proximal region of a neurological stimulation lead. Lead extension connector 36 includes a lead receiving port 54 for receiving the proximal region of a neurological stimulation lead.

FIG 3 shows in more detail the band stop filter of FIG 2 having first band stop filter 50 coupled to first conductor 48 and a second band stop filter 44 coupled to second conductor 42. First band stop filter 50 is coupled in series between first conductor 48 and the portion of the first conductor leading to the first lead electrode as indicated at 51. Similarly, second band stop filter 44 is coupled in series with second conductor 42 and the second conductor portion leading to the second lead electrode as indicated at 45.

In some embodiments, each and every conductor wire within the neurological stimulation lead extension includes a band stop filter. First band stop filter 50 is shown having a capacitor indicated by C in series with a non-ideal resistance indicated by R_{C}. Similarly, the band stop filter inductor is indicated by an L and is in series with the non-ideal resistance of the conductor indicated by R_{L}. The capacitor and inductor are coupled in parallel to form a so-called tank circuit or band stop filter. By selecting the proper values of the inductor and the capacitor, along with careful attention to the non-ideal real properties of the capacitor and the inductor, a band stop filter can be created as is well known now to those skilled in the art. At a frequency and wavelength expected from a typical MRI system, the band stop filters can be engineered to resonate at that frequency, creating a very high impedance and effectively breaking the electrical continuity of the lead, thereby shortening the effective length of the lead and substantially changing the RF resonant properties of the lead.

In some embodiments, the value of L is between about 1 and 100 nanohenries. The value of C may be between about 100 to 4000 picofarads.

MRI systems vary in static field strength from 0.5 Tesla all the way up to 3 Tesla with newer research machines going much higher. This is the force of the main static magnetic field. The frequency of the pulsed RF field associated with MRI is found by multiplying the static field in Teslas times 42.45. Accordingly, a 3 Tesla MRI system has a pulsed RF field of approximately 128 MHz.

The Q factor can be expressed using the equation Q= = fᵣ / Δ f_{3dB}, where fᵣ is the resonance frequency and Δ f_{3dB} is the bandwidth of the BSF. The bandwidth of the BSF can be taken as the difference between the two measured frequencies on either side of fᵣ at the 3dB loss points as measured on an insertion loss chart. The resonance frequency is the average between these two frequencies on either side. As can be seen in this relationship, higher Q values result in a narrower 3 dB bandwidth.

In some BSFs, the Q of the inductor is relatively high and the Q of the capacitor is relatively low to select the overall Q of the band stop filter. The inductor can have a relatively low resistive loss and the capacitor has a relatively high equivalent series resistance. The overall Q of the band stop filter can be selected to attenuate current flow through the lead wire along a range of selected frequencies. In some embodiments, the range of selected frequencies includes a plurality of MRI pulsed frequencies.

If a lead electrode were located to the right of reference numeral 51 in FIG 3, and if the length of conductor shown was at a resonant frequency of the MRI system, the absorbed RF energy could generate heat at the distal electrodes. With band stop filter 50 in place, the effective length of the wire is shortened and the electrical continuity from the proximal portion of the wire to the left of the lead electrode is also disrupted by the greatly increased impedance of band stop filter 50 at the MRI frequency. While it would be ideal to have a band stop filter located deep in the brain near the distal electrode, as previously explained, this itself is less than desirable. Applicants have determined that while implanting band stop filters within the brain may not be feasible for most applications, locating the band stop filters soon after the lead exits the body can also be effective in substantially changing the effective length of the conductor and breaking the electrical continuity between the more proximal conductor regions and the more distal regions leading to the implanted distal electrode.

FIG 4 shows another aspect of the present invention. A lead 70 is shown having a distal region 72, a proximal region 74, and an intermediate region 75 located in between the distal and proximal regions. In some embodiments, intermediate region 75 is located about halfway between the distal and proximal regions. In other embodiments, intermediate region 75 is located such that the distance between the proximal most electrical contact and the proximal end is greater than the distance from the proximal most electrical contact to the lead distal end. Distal region 72 includes a first electrode 76 and a second electrode 78. Intermediate region 75 includes a first contact 77 and a second contact 79. Electrodes 76 and 78 can be used for neurological stimulation, while contacts 80 and 82 can be used to couple to a lead extension.

A severing location 73 is also shown. In various embodiments, severing location 73 can include some visual indicia of the location, and/or a region of preferential weakness. In various embodiments, the region of preferential weakness can include circumferential scoring and/or perforations to promote severing at the desired severing location 73. In use, and with possible reference to FIG 1, lead 70 can be advanced through a cannula to the target site in the brain, spinal epidural space, or other tissue. Intermediate region 75 and proximal region 74 may both extend proximally out of the cannula. Once the cannula is removed over lead 70, the lead can be severed proximal of the proximal most electrical contact, for example, at indicated severing location 73. In some embodiments, not requiring separate illustration, a set of corresponding proximal electrical contacts similar to contacts 77 and 79 can be provided in proximal region 74. Such contacts can be used to stimulate and be in electrical communication with the distal electrodes 76 and 78. Such electrode contacts in the proximal region could be used during the implantation process.

But for the severing of the lead, any lead extension would likely be coupled to the lead proximal region 74 as shown, and the length of the entire lead would be at least about twice the length of the implanted portion of the lead. This is because the lead must often be double the required length just to allow removing the cannula over the lead while maintaining control of the lead. Severing lead 70 can thus be used to attach band stop filters at a location much closer to the distal electrodes than would otherwise be possible. By shortening the lead in this way, the effective length of the RF antenna can be shortened and the RF energy absorbed by the lead extension length proximal of the band stop filters decoupled from the distal electrodes.

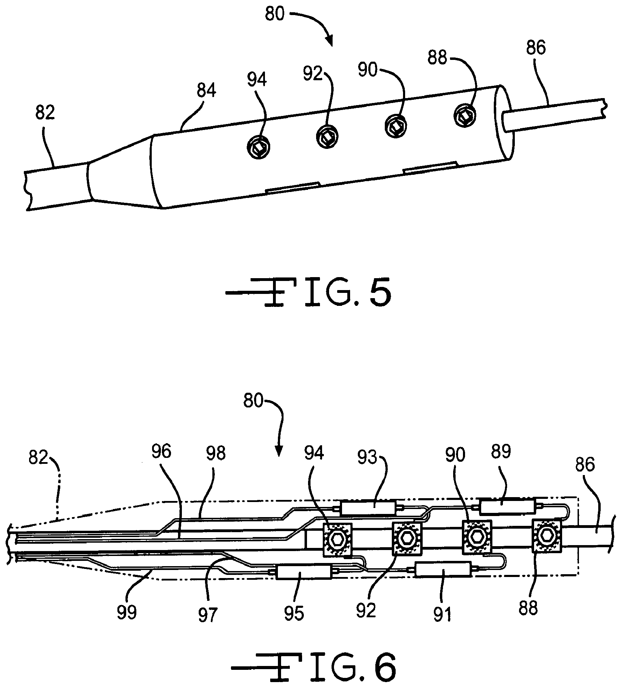

FIG 5 illustrates one lead extension distal connector 80 according to the present invention. Connector 80 includes generally a body 84 and extends to a lead extension distal region 82. The lead extension connector 80, in the example shown, has received a lead proximal region 86 carrying four contacts (not shown in FIG 5). Each of the four lead electrical contacts can be contacted by the four connector block screws, indicated at 88, 90, 92, and 94. Devices for coupling lead electrode contacts to lead extenders are well-known in the art. Various devices including devices requiring tools to secure as shown in FIG 5 and tool-less receiving contacts are also well known. In some embodiments, the lead extension distal connector can be simply a connector, with the band stop filters located proximal of the connector. In the embodiment illustrated, the band stop filters are incorporated as part of the lead extension distal connector, further illustrated in FIG 6.

FIG 6 further illustrates lead extension connector 80. Lead 86 has been inserted and secured by the four connector blocks 88, 90, 92, and 94, as previously described. The lead extension distal region 82 may be seen at left. The four lead electrical contacts are secured within the respective connector blocks. A first band stop filter 89 is coupled to first connector block 88 and extends through a first connector wire 96 to the remainder of the lead extension. A second band stop filter 91 is coupled to the second connector block 90 and extends through a second conductor wire 97 to the remainder of the lead extension body. A third band stop filter 93 is coupled to the third connector block 92 and extends through a third conductor wire 98 to the lead extension body. A fourth band stop filter 95 is coupled to the fourth connector block 94 and extends through a fourth conductor wire 99 to the remainder of the lead extension. As previously discussed, the connector blocks are replaced by tool-less connectors in some embodiments.

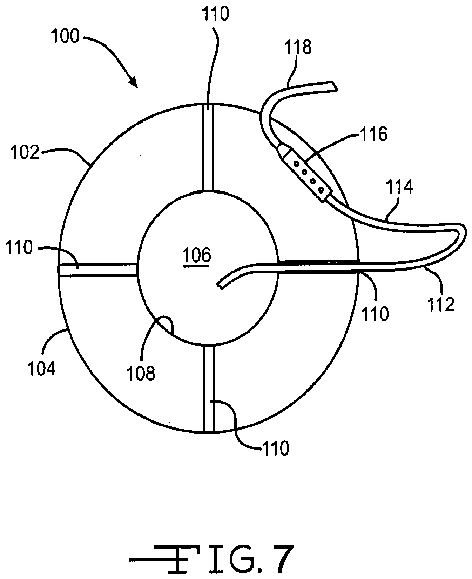

FIG 7 illustrates a burr hole cap base 100 according to the present invention. Burr hole caps and bases are well known to those skilled in the art and are described in U.S. Patent Nos. 4,328,813; 5,464,446; and 5,927,277 and U.S. Published Application No. 2007/0233158. Base 100 has an annular shape having a central opening or aperture 106, an inner periphery 108, an outer periphery 104, and a body 102. Several lead engaging, dressing, or fixation grooves 110 are shown in base 100. Some embodiments have only one such engaging structure while others have more than one. In some embodiments, the width of the groove narrows as the lead extends outward from a central aperture 106. In this way, the lead can be dressed, engaged, and effectively fixed with respect to translation within respect to base 102. Lead engaging and fixation mechanisms and structures for burr hole caps are well-known in the art. In the embodiment illustrated, a lead extension distal connector 116 is coupled to the base body 102. A portion of lead extension 118 is also shown, extending proximally from lead extension connector 116. In use, deep brain stimulation lead 112 exits the burr hole through central aperture 106 and extends outward through a lead engaging structure 110. The excess lead length may be dressed in various ways in various embodiments. In some embodiments, the base body may be rotated to dress excess lead around the periphery. In other embodiments, the lead engaging groups or structures may be selected from more than one in a location to take up a sufficient amount of excess lead. In many embodiments, lead extension 118 does not extend across the burr hole central aperture.

As shown in FIG 7, the lead proximal region 114 carrying the electrical contacts can be inserted into the lead extension connector 116. With the lead proximal region thus secured, electrical continuity with the lead extension is obtained. In some embodiments, the band stop filters are included within the lead extension distal connector as previously described. In other embodiments, the band stop filters can be spread out more within the base body 102 as the space constraints may not be as great in the base as within the connector. Specifically, the connector blocks can be located further from the band stop filters if that is desired. In still other embodiments, the band stop filters can be located in and proximally further down the lead extension body. In some methods, the distance between the band stop filters and the distal region of the stimulation lead is selected to not be at a resonant or strong resonant wavelength of the expected MRI system.

## Claims

1. A lead extension (36; 80; 118) for coupling to a lead (26, 70), the lead (26; 70) including a lead distal region (28; 72) having at least one electrode thereon, a lead proximal region (34; 74; 86) having at least one electrical contact thereon, and a lead length, the lead extension (36; 80; 118) comprising: a lead extension body (40; 84) having a lead extension proximal region, a lead extension distal region (38; 82), and at least one electrical conductor disposed within and extending between the lead extension proximal and distal regions; at least one band stop filter (BSF) (44, 50; 89, 91, 93, 95) including a capacitor (C) in parallel with an inductor (L), the parallel capacitor and inductor (C, L) combination placed in series with each of the electrical conductors somewhere between the lead extension proximal and distal regions, wherein values of capacitance and inductance have been selected such that the band stop filter is resonant around a selected frequency; **characterised in that** the lead extension (36; 80; 118) includes a distal connector and in which the BSF is included within the lead extension distal connector, wherein the lead extension distal connector includes at least one insertion port (54) for receiving the lead proximal region (34; 74; 86) and in which the BSF is coupled to the insertion port (54).

2. The lead extension (36; 80; 118) of claim 1, wherein the parallel capacitor and inductor (C, L) combination are placed in series with each and every one of the electrical conductors somewhere between the lead extension proximal and distal regions.

3. The lead extension (36; 80; 118) of claims 1 to 2, wherein the BSF has a Q factor, and the overall Q factor of the BSF is selected to balance impedance at the selected frequency versus frequency band width characteristics.

4. The lead extension (36; 80; 118) of claims 1 to 3, wherein the BSF is located a distance from the lead extension distal region (38; 82) adapted to reduce resonance with the MRI signal when used in combination with the lead length, preferably wherein the distance from the BSF to the lead distal end (28; 72), when the lead (26; 70) and the lead extension (36; 80; 118) are coupled together, is substantially less than a half-wavelength of an expected MRI system selected from the group consisting of 1.5 and 3 Tesla MRI systems, more preferably wherein the distance from the BSF to the lead distal end (28; 72) when the lead (26; 70) and the lead extension (36; 80; 118) are coupled together is less than about 15 cm.

5. The lead extension (36; 80; 118) of claims 1 to 4, wherein the lead extension distal region (38; 82) includes a burr hole cap base (100) for capping a burr hole (30) , the burr hole cap base (100) including: an annular body (102) disposed between a central aperture (106) and an outer periphery (104), the body (102) being MRI compatible; at least one connector coupled to the body (102) for electrically coupling to the lead proximal contact; and in which the BSF is mechanically coupled to the burr hole cap base (100).

## Patentansprüche

1. Leiter-Verlängerung (36; 80;118) zum Koppeln an einen Leiter (26, 70), wobei der Leiter (26; 70) einschließt: einen distalen Leiter-Bereich (28; 72), welcher wenigstens eine Elektrode daran aufweist; einen proximalen Leiter-Bereich (34; 74; 86), der wenigstens einen elektrischen Kontakt daran aufweist, und eine Leiter-Länge; wobei die Leiter-Verlängerung (36; 80; 118) umfasst: einen Leiter-VerlängerungsKörper (40; 84), der einen proximalen Leiter-Verlängerungs-Bereich, einen distalen Leiter-Verlängerungs-Bereich (38; 82) und wenigstens einen elektrischen Leiter aufweist, der angeordnet ist innerhalb und sich erstreckt zwischen den proximalen und distalen Leiter-Verlängerungs-Bereichen; wenigstens ein Band-Stop-Filter (BSF) (44, 50; 89, 91, 93, 95), das einen Kondensator (C) parallel mit einem Induktor (L) einschließt, wobei die Kombination aus parallelem Kondensator und Induktor (C, L) in Reihe platziert ist mit jedem der elektrischen Leiter irgendwo zwischen den proximalen und distalen Bereichen der Leiter-Verlängerung, wobei Werte der Kapazitanz und Induktanz derart gewählt wurden, dass das Band-Stop-Filter resonant um eine gewählte Frequenz ist, **dadurch gekennzeichnet, dass** die Leiter-Verlängerung (36; 80; 118) einen distalen Konnektor einschließt und in dem das BSF in dem distalen Leiter-Verlängerungs-Konnektor eingeschlossen ist, wobei der distale Leiter-Verlängerungs-Konnektor wenigstens eine Einsetz-Öffnung (54) zum Aufnehmen des proximalen Leiter-Bereichs (34; 74; 86) einschließt und in der das BSF mit der Einsetz-Öffnung (54) gekoppelt ist.

2. Leiter-Verlängerung (36; 80; 118) nach Anspruch 1, wobei die Kombination aus parallelem Kondensator und Induktor (C, L) in Reihe platziert ist mit jedem und jedem einzelnen der elektrischen Leiter irgendwo zwischen den proximalen und distalen Leiter-Verlängerungs-Bereichen.

3. Leiter-Verlängerung (36; 80; 118) nach den Ansprüchen 1 bis 2, wobei das BSF einen Q-Faktor aufweist und der Gesamt-Q-Faktor des BSF so gewählt ist, dass er die Impedanz bei den gewählten charakteristischen Werten der Frequenz gegen die Frequenz-Bandbreite ausgleicht.

4. Leiter-Verlängerung (36; 80; 118) nach den Ansprüchen 1 bis 3, wobei das BSF in einer Entfernung von dem distalen Leiter-Verlängerungs-Bereich (38; 82) angeordnet ist, die dafür angepasst ist, eine Resonanz mit dem MRI-Signal zu reduzieren, wenn es in Kombination mit der Leiter-Länge (26; 70) verwendet wird; vorzugsweise worin die Entfernung von dem BSF zu dem distalen Leiter-Ende (28; 72) dann, wenn der Leiter (26; 70) und die Leiter-Verlängerung (36; 80; 118) zusammengekoppelt werden, wesentlich geringer ist als eine Halb-Wellenlänge eines erwarteten MRI-Systems, das gewählt ist aus der Gruppe, die besteht aus 1,5- und 3-Tesla-MRI-Systemen; noch mehr bevorzugt worin die Entfernung von dem BSF zu dem distalen Leiter-Ende (28; 72) dann, wenn der Leiter (26; 70) und die Leiter-Verlängerung (36; 80; 118) zusammengekoppelt werden, geringer ist als etwa 15 cm.

5. Leiter-Verlängerung (36; 80; 118) nach den Ansprüchen 1 bis 4, wobei der distale Leiter-Verlängerungs-Bereich (38; 82) eine Bohrloch-Verschluss-Basis (100) zum Verschließen eines Bohrlochs (30) einschließt, wobei die Bohrloch-Verschluss-Basis (100) einschließt: einen ringförmigen Körper (102), der zwischen einer zentralen Öffnung (106) und einem Außenumfang (104) angeordnet ist, wobei der Körper (102) MRI-kompatibel ist; wenigstens ein Verbindungselement an dem Körper (102) zum elektrischen Koppeln mit dem proximalen Leiter-Kontakt gekoppelt ist; und in dem das BSF mechanisch mit der Bohrloch-Verschluss-Basis (100) gekoppelt ist.

## Revendications

1. Rallonge de câble (36 ; 80 ; 118) destinée à être raccordée à un câble (26,70), le câble (26 ; 70) incluant une partie distale du câble (28 ; 72) comportant au moins une électrode, une partie proximale du câble (34 ; 74 ; 86) comportant au moins un contact électrique, et une longueur de câble, la rallonge de câble (36 ; 80 ; 118) comprenant : un corps de rallonge de câble (40 ; 84) possédant une partie proximale de rallonge de câble, une partie distale de rallonge de câble (38 ; 82), et au moins un conducteur électrique disposé à l'intérieur et s'étendant entre les parties proximale et distale de la rallonge de câble ; au moins un filtre stop bande (BSF) (44, 50 ; 89, 91, 93, 95) incluant un condensateur (C) connecté en parallèle avec un inducteur (L), la combinaison du condensateur et de l'inducteur (C, L) disposés en parallèle étant placée en série avec chacun des conducteurs électriques quelque part entre les extrémités proximale et distale de la rallonge de câble, où les valeurs de capacitance et d'inductance ont été sélectionnées de manière à ce que le filtre stop bande résonne autour d'une fréquence choisie ; **caractérisé en ce que** la rallonge de câble (36 ; 80 ; 118) inclut un connecteur distal et dans laquelle le filtre stop bande (BSF) est inclus à l'intérieur du connecteur distal de la rallonge de câble, où le connecteur distal de la rallonge de câble inclut au moins un orifice d'insertion (54) destiné à recevoir la région proximale du câble (34 ; 74 ; 86) et dans lequel le filtre stop bande (BSF) est couplé à l'orifice d'insertion (54).

2. Rallonge de câble (36 ; 80 ; 118) selon la revendication 1, au sein de laquelle le condensateur et l'inducteur (C, L) combinés en parallèle sont placés en série avec chacun des conducteurs électriques sans exception quelque part entre les extrémités proximale et distale de la rallonge de câble.

3. Rallonge de câble (36 ; 80 ; 118) selon les revendications 1 à 2, où le filtre stop bande (BSF) comporte un facteur Q, et le facteur Q global du filtre stop bande (BSF) est sélectionné de façon à équilibrer l'impédance à la fréquence sélectionnée par opposition aux caractéristiques de la largeur de bande de fréquence.

4. Rallonge de câble (36 ; 80 ; 118) selon les revendications 1 à 3, où le filtre stop bande (BSF) est situé à une distance de la partie distale de la rallonge de câble (38 ; 82) adaptée de manière à réduire la résonance avec le signal IRM lors de l'utilisation combinée avec la longueur de câble, où la distance entre le filtre stop bande (BSF) et l'extrémité distale du câble (28 ; 72), lorsque le câble (26 ; 70) et la rallonge du câble (36; 80 ; 118) sont couplées, est substantiellement inférieure à une demi-longueur d'onde d'un système d'IRM attendu choisi parmi le groupe comprenant des systèmes IRM Tesla 1.5 et 3, où la distance entre le filtre stop bande (BSF) et l'extrémité distale du câble (28 ; 72), lorsque le câble (26 ; 70) et la rallonge du câble (36 ; 80 ; 118) sont couplées, est plus préférablement inférieure à environ 15 cm.

5. Rallonge de câble (36 ; 80 ; 118) selon les revendications 1 à 4, où la partie distale de la rallonge de câble (38 ; 82) inclut une base de capuchon pour orifice de trépanation (100) pour recouvrir un orifice de trépanation (30), la base de capuchon pour orifice de trépanation (100) incluant : un corps annulaire (102) situé entre l'ouverture centrale (106) et une périphérie externe (104), le corps (102) étant compatible à l'IRM ; au moins un connecteur couplé au corps (102) pour le couplage électrique avec le contact proximal du câble ; et dans lequel le filtre stop bande (BSF) est couplé mécaniquement à la base de capuchon pour orifice de trépanation (100).
